# EUROPEAN PATENT SPECIFICATION

(11) **EP 4 725 023 B1**
(45) Date of publication and mention of the grant of the patent: **18.11.2026**
(21) Application number: 24728656.0
(22) Date of filing: 31.05.2024
(51) Int. Cl.: G16H 10/60, G06F 21/62

(54) **DISTRIBUTED CLINICAL DATA MANAGEMENT SOLUTION WITH PATIENT ID PROTECTION**
VERTEILTE KLINISCHE DATENVERWALTUNGSLÖSUNG MIT PATIENTEN-ID-SCHUTZ
SOLUTION DE GESTION DE DONNÉES CLINIQUES DISTRIBUÉES AVEC PROTECTION DE L'ID DU PATIENT

(30) Priority: 09.06.2023 WO PCT/CN2023/099324; 19.07.2023 EP 23186420
(43) Date of publication of application: 15.04.2026
(73) Proprietor: Koninklijke Philips N.V., 5656 AE Eindhoven (NL)
(72) Inventor: ZHANG, Jinsheng, 5656 AG Eindhoven (NL); CHEUNG, Yee Him, 5656 AG Eindhoven (NL)
(74) Representative: Philips Intellectual Property & Standards
(86) International application number: PCT/EP2024/065100
(87) International publication number: WO 2024/251627

(56) References cited:
- US-A1- 2021 104 304

## Description

### FIELD OF THE INVENTION

This invention relates to a patient data directory system, a patient device for use by a patient, a data provider device for use by a data provider, a data requestor device, a method of managing access to clinical records of a patient, and a non-transitory computer-readable medium comprising a program that, when executed by a processing system, causes the processing system to perform the method.

### BACKGROUND OF THE INVENTION

One of the biggest problems faced by the healthcare industry is the efficient management and prompt access to a patient's clinical data residing on multiple heterogeneous medical systems. With clinical data scattered over different medical sites, the patient typically does not have a complete view of his/her medical history. Additionally, due to lack of full control over these medical records, the patient is unaware of how the clinical data in the records are utilized. In like manner, it is difficult for clinicians to make correct and informed decisions without a full picture of the patient's medical profile.

Conventionally, a patient's record at each medical site may be uploaded to a 'cloud' server, and accessed as required by clinicians having access to the server. However, such a consolidation of records from a variety of medical sites incurs substantial overhead at the sites, typically requiring a transformation of the data at each site to conform to the requirements of the cloud storage facility with regard to the structure and format of the information being stored, and requires a patient identifier that is common to each of the medical sites. Typically, the common patient identifier is an identifier that is unique to the patient, such as the patient's National ID or Social Security Number. Additionally, to avoid the risk of a 'single point of failure', multiple redundant storage facilities must be provided.

Of particular concern, a data breach at a consolidated data storage facility may result in the unauthorized release of the patient's identity and the patient's entire medical history.

US 2021/104304 A1 describes a system and method that utilizes private key encryption and public and private keys for secure transmissions, which is said to enable the patients to control access to their own medical records in a secure Internet cloud storage, and also to grant doctors, hospitals, pharmacies and other medical and health care service providers "on purpose" or "on demand" limited and temporary access to the patients' medical records when needed.

### SUMMARY OF THE INVENTION

The present invention is defined in the independent claims. The embodiments are defined in the dependent claims. The examples, aspects and embodiments not falling within the scope of the claims are for reference only.

Since
clinical data contain highly sensitive information, a solution is required that enables linking the medical records of the patient at the multiple medical sites, while at the same time protecting the patient's privacy to the greatest extent possible.

To better address one or more of these concerns, it would be advantageous to provide a solution that enables each medical site to locally store the patient's clinical data using a locally defined identifier of the patient, preferably a site-specific pseudo-ID. Although each site must maintain a mapping of the local pseudo-ID to the actual identity of the patient, this mapping may be stored securely in a database that is independent of the database that is used to store the clinical data. Additionally, because each medical site assigns a local pseudo-ID to the patient, a data breach of the pseudo-ID to actual patient identity mapping at one site will not affect the security of the patient's identity, or the security of the clinical data, at any other site.

The invention provides for a patient data directory system, a patient device for use by a patient, a data provider device for use by a data provider, a data requestor device, a computer implemented method, and a non-transitory computer-readable medium as defined in the claims. Embodiments are given in the dependent claims.

Each medical site stores the patient's clinical data in a local database, indexed by the patient's site-specific pseudo-ID.

To facilitate a linking of the patient's clinical data among each of the medical sites that contain the patient's clinical data, a Patient Data Directory System (PDDS) is provided. As each medical site stores the patient's clinical data, a set of metadata is provided that identifies where the clinical data is stored at the medical site, as well as other information, such as the patient's pseudo-ID at the particular medical site, that facilitates retrieval of this clinical data, and other data that serves to indicate what information is contained in the clinical data, such as a medical code that identifies the anatomical region related to the clinical data. This metadata is entered into the PDDS, indexed by a patient-selected identifier, such as a PIN.

The metadata includes the URL (Universal Record Locator) of the particular site, the site-specific pseudo-ID of the patient, and other data as required to facilitate retrieval of the clinical data, such as cryptographic keys.

The patient controls the access to and retrieval of the metadata, thereby controlling what information is released to any particular clinician. In an embodiment, the clinician that generates and stores the clinical data at a site provides the metadata to the patient, and the patient stores the metadata in the PDDS. When a clinician has a need for the patient's clinical data at other medical sites, the clinician contacts the patient, the patient selectively provides the metadata related to the clinician's request, e.g., the metadata relating to a selected set of records of the patient, and the clinician or in general the data requestor retrieves the clinical data at the other sites using this metadata. The retrieved clinical data may thus be the data associated with the selected set of records. In another embodiment, the metadata of all records of the patient may be provided to the data requestor.

The identity of each clinician is authenticated, and an Authentication Token is issued to the clinician upon successful authentication. This Authentication Token is used to identify the clinician to the clinical sites, and to determine whether the authenticated clinician is authorized to gain access to the clinical data stored at each site that is identified by the metadata provided by the patient. When the patient first goes to a clinician, the clinician also uses the Authentication Token to register the patient and initiate the patient's access to the PDDS.

The patient data directory system (PDDS) comprises a plurality of records associated with a patient, and an access control system that controls access to the plurality of records, wherein access to the plurality of records is controlled by the patient. The patient can approve or deny access to the clinical data of the patient by each of a plurality of data requestors. The plurality of records comprises metadata that is associated with the clinical data of the patient that is stored at a plurality of clinical sites. Each clinical site identifies the patient using a site-specific pseudo-ID (SPID) of the patient, and the metadata of each record identifies a location of the patient's clinical data at a corresponding clinical site, and the SPID of the patient at the corresponding clinical site. The access control system comprises an identity verification process to verify that a user of the system is the patient that controls access to the metadata records. If the user is verified as being the patient, and data access is approved for the data requestor, the metadata is communicated to the data requestor to enable the data requestor to access to the clinical data of the patient at the corresponding clinical site based on the SPID of the patient at the corresponding clinical site. As detailed further below, the PDDS may communicate the metadata to the patient, and the patient forwards the metadata to the data requestor.

A patient device comprises a communication system for communicating with a data requestor and the PDDS. The patient device includes a storage system, a cryptographic system, and a user interface. In use, the patient device receives a record-update request from the data provider and presents the request to the patient for approval or denial. The patient device uses the cryptographic system to gain access to the PDDS, and uses the communication system to communicate the metadata to the PDDS.

The patient device subsequently receives a record-access request from a data requestor and presents the request to the patient for approval or denial. If the request is approved, the patient device uses the cryptographic system to gain access to the metadata records of the patient at the PDDS, and, in response to the record-access request, receives the metadata records of the patient from the PDDS. The patient device selectively communicates some or all of the metadata to the data requestor, to facilitate access to the clinical data of the patient stored at the plurality of clinical sites by the data requestor.

A data provider device comprises a communication system for communicating with a patient, the PDDS, and a clinical site server associated with the data provider. The data provider device stores the patient's clinical data in a clinical site server and receives corresponding metadata from the server in response. The data provider device communicates patient verification information to the PDDS if the patient is new to the PDDS, and communicates a record-update request (metadata storage request) including the metadata to the patient for subsequent submission to the PDDS.

A data requestor device comprises a communication system for communicating with a patient and a plurality of clinical sites. The data requestor device communicates a data access request to the patient, and receives the metadata associated with the clinical data from the PDDS via the patient. The data requestor device subsequently accesses the clinical data of the patient stored at the plurality of clinical sites based on the received metadata.

Additionally, this invention includes a computer implemented method of managing access to clinical records of a patient, comprising: storing a plurality of records that facilitate access to the clinical records of the patient, wherein the plurality of records comprises metadata that is associated with clinical data of the patient that is stored at a plurality of clinical sites, wherein the metadata of each record comprises an identification of a clinical site of the plurality of clinical sites and a site-specific pseudo-ID (SPID) associated with the patient at the clinical site, wherein the site-specific pseudo-ID is necessary to access the clinical data at the clinical site, receiving a record access request from a user, verifying that the user is the patient that controls access to the metadata records, if the user is verified to be the patient and access to the clinical data of the patient is approved for a data requestor by the patient, providing the metadata requested in the record access request to the patient; and communicating the metadata from the patient to the data requestor to enable the data requestor to access the clinical data of the patient at the corresponding clinical site based on the SPID of the patient at the corresponding clinical site.

### BRIEF DESCRIPTION OF THE DRAWINGS

The invention is explained in further detail, and by way of example, with reference to the accompanying drawings wherein:
FIG. 1 illustrates an overview of the architecture of an example embodiment of this invention.
FIG. 2 illustrates an example of a clinician authentication system.
FIG. 3 illustrates an example message exchange for initiating a patient's access to the PDDS.
FIG. 4 illustrates an example flow diagram for reading or writing metadata on the PDDS.
FIG. 5 illustrates an example flow diagram for providing the patient's clinical data to the clinician, based on the patient's metadata from the PDDS.
FIG. 6 illustrates an example flow diagram for enabling the re-encryption of records in the PDDS.
FIG. 7 illustrates a flow diagram of an example method for providing access to a patient's metadata records that are stored at the PDDS.

Throughout the drawings, the same reference numerals indicate similar or corresponding features or functions. The drawings are included for illustrative purposes and are not intended to limit the scope of the invention.

### DETAILED DESCRIPTION

In the following description, for purposes of explanation rather than limitation, specific details are set forth such as the particular architecture, interfaces, techniques, etc., in order to provide a thorough understanding of the concepts of the invention. However, it will be apparent to those skilled in the art that the present invention may be practiced in other embodiments, which depart from these specific details. In like manner, the text of this description is directed to the example embodiments as illustrated in the Figures, and is not intended to limit the claimed invention beyond the limits expressly included in the claims. For purposes of simplicity and clarity, detailed descriptions of well-known devices, circuits, and methods are omitted so as not to obscure the description of the present invention with unnecessary detail.

For ease of understanding, the invention presented herein is presented in the context of a patient data directory that is controlled by the patient to facilitate access by clinicians to clinical data related to the patient. One of skill in the art will recognize, however, that the principles of this invention can be applied to other environments and applications.

Accordingly, the terms 'patient', 'clinician', 'clinical data', etc. are used in this context, but are herein defined to be generic terms for 'first participant', 'second participant', 'data associated with the first participant', etc. In like manner, the term 'patient' is defined herein to include a party that is authorized by the patient/'first participant' to access the data directory on behalf of the patient/'first participant', and the term 'clinician' is defined herein to include a party that is authorized to access the clinical data associated with the patient/'first participant'.

One of skill in the art will also recognize that the actions of the patient, the clinician, the site, etc. described herein will generally be performed using a device configured to perform the actions of the patient, a device configured to perform the actions of the clinician, a device/server configured to perform the actions of the site, etc. Thus, the terms 'patient' and 'patient device'; 'clinician' and 'clinician device; 'site' and 'site server', etc. may be used interchangeably herein. Similarly, a clinician may be a 'data provider' who provides the metadata that is to be stored using a 'data provider device', or a 'data requestor' who requests and receives the stored metadata using a 'data requestor device'. The data provider device and data requestor device may be embodied as a single component.

In like manner, one of skill in the art will recognize that the particular cryptographic methods presented in the example embodiments may be replaced by other cryptographic methods. For example, where feasible, a disclosed symmetric encoding method may be replaced by an asymmetric encoding method to encrypt the material to be protected from disclosure, and vice versa.

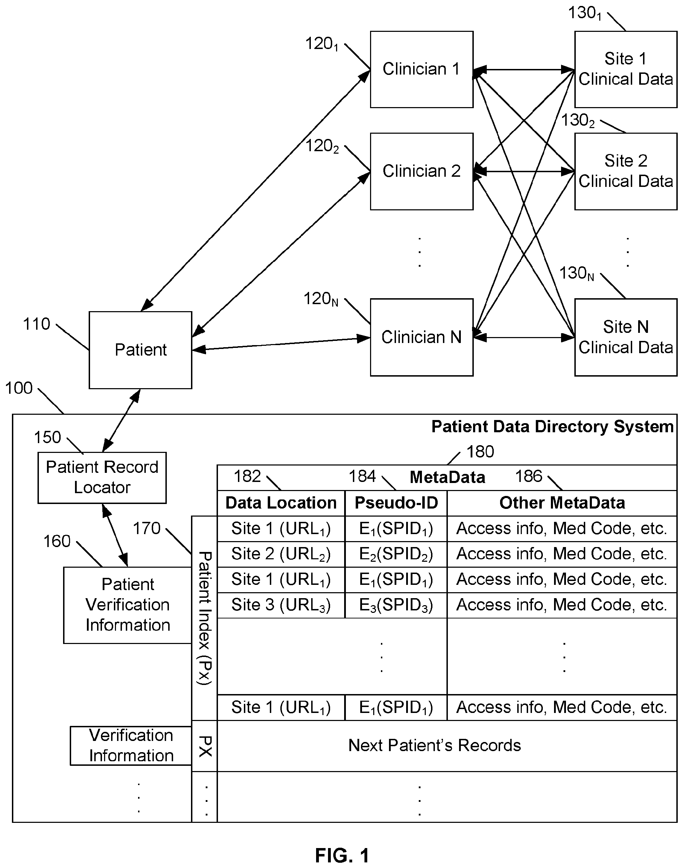

FIG. 1 illustrates an overview of the architecture of an example embodiment of this invention. Over time, a patient 110 may visit multiple clinicians 120₁, 120₂, ... 120_{N}, each of these clinicians being associated with a particular medical site. The clinicians store the patient's clinical data 130₁, 130₂, ... 130_{N} at the corresponding medical site. Although FIG. 1 illustrates a one-to-one correspondence between clinicians and sites for ease of understanding, one of skill in the art will recognize that multiple clinicians may be associated with each site. In like manner, each medical site is illustrated as having a single set of clinical data associated with the patient, whereas one of skill in the art would recognize that a medical site may have a variety of different departments, each having clinical records associated with the patient. As used herein, the term 'site' includes 'sub-sites', such as the different departments at a site. Also for ease of understanding, the terms 'clinician' and 'site' may be used interchangeably when referring to actions taken at each site.

To preserve the patient's privacy, each site (or sub-site) identifies the patient using a pseudo-ID, such as a patient number or other identifier associated with the patient's clinical records, and access to the patient's clinical data requires knowledge of that patient's pseudo-ID. Typically, a patient will identify himself/herself to the clinician, and on the first visit to the medical site, the medical site will assign a pseudo-ID for this site to the patient. Thereafter, the clinician will identify the patient's clinical data using the site-assigned pseudo-ID corresponding to the patient. Note that each site (or sub-site) may assign a different pseudo-ID to the patient.

When the clinician enters the patient's clinical data as a record in the site's storage facility, the clinician, or the site, creates 'metadata' that is associated with the record. In particular, the metadata includes an identification of the record that facilitates access to the record, such as the URL of the particular site, and, optionally, an indication of the location of the record at this URL. Additionally, because the patient's pseudo-ID at the site is required to access the patient's clinical data at the site, the metadata associated with the record also includes the patient's pseudo-ID at this particular site. Preferably, the site-specific pseudo-ID of the patient is encrypted by the site, and this encrypted pseudo-ID is included in the metadata. In this manner, the site-specific pseudo-ID of the patient is known only to the particular site.

Additional metadata may include information that characterizes the clinical data that is stored at the record, such as an identification of the anatomic region, a classification of the illness being treated, the date of treatment, and so on. In some embodiments, a set of pre-defined medical 'codes' may be used to classify the content of the record. This additional metadata may be used to 'filter' access to the record. For example, the user may grant a clinician access to only those records related to a particular anatomic region, or records related to a particular range of dates, and so on.

This metadata for each record of the patient's clinical data at each site is stored in a Patient Data Directory System (PDDS) 100. The PDDS 100 may be used to store the metadata of multiple patients, and the PDDS 100 includes a patient record locator 150 that identifies the particular patient based on a set of patient verification information 160, discussed further below. As illustrated in FIG. 1, the metadata 180 of each record includes a data location 182, such as the site's URL and, optionally, an indication of where, at the site, the record is located. The metadata also includes the site's pseudo-ID (SPID) 184 assigned to the patient, preferably encrypted by the site, and other metadata 186. The other metadata 185, as discussed above, includes any further information required to access the record, as well as information that characterizes the content of the record, such as a medical code.

One of skill in the art will recognize that the particular data structure of the metadata 180 at the PDDS 100 may differ from the linear structure illustrated in FIG. 1. For example, the data structure may be hierarchical, with the site information (site location 182 and SPID 184) at a higher level, and each of the specific 'other' metadata 186 associated with each new set of clinical data associated with the patient at this site being 'sub-records' below the site location information. Other structures, including linked-lists and others, may also be used.

Each time a new set of clinical data 130 for the patient 110 is stored at a site, the metadata 180 related to this clinical data is stored at the PDDS 100, indexed by an identification (Px) 170 of the particular patient. In the example of FIG. 1, the metadata 180 for the patient 110 indicates that the patient initially visited Site₁, then visited Site₂, then revisited Site₁, then visited Site₃, etc.

In embodiments of this invention, access to the metadata stored at the PDDS is controlled by each patient. At the patient's first visit to a first clinician, the first clinician 'registers' the patient at the PDDS 100, and in conjunction with the patient 110, provides the patient verification information 160 to facilitate secure access to the metadata 180 that is subsequently added to the patient's record at the PDDS 100.

Because the patient verification information 160 is designed to use secure techniques for granting access to only verified patients, the patient is able to maintain control of the storage and retrieval of the patient's metadata. The patient verification information 160 may include one or more keys used to represent the patient in the PDDS and to link each of the patient's plurality of records. A privacy-preserving cryptographic protocol based on the one or more multiple keys is used by the PDDS to limit access to the identified patient. The one or multiple keys may comprise one or multiple random keys generated by the patient and stored in PDDS. Preferably, the one or more multiple keys do not reveal identification information of the patient.

In some embodiments, the privacy-preserving cryptographic protocol may require a one-time setup and subsequent challenge-response authentications. The one-time setup may require the patient to generate a public key and a private key of a public/private key pair. The private key is stored by the patient and the public key is stored in the PDDS, preferably by the clinician when the patient is first registered at the PDDS.

As illustrated in FIG. 1, each clinician 120 may access records at multiple sites 130, provided that the patient provides the metadata of each accessible site to the clinician. Typically, each clinician has read and write access to the site that the clinician is associated with, as indicated by the double-head arrows between each clinician and the associated site. Each clinician typically has read-only access to the sites that the clinician is not associated with, as indicated by the single-head arrow between each clinician and each non-associated site. As detailed further below, each clinician that is authorized to access information at the clinical sites is provided an authorization token, and provides the authorization token to each specific clinical site to gain access and retrieve the clinical data of the patient at each specific clinical site.

When a clinician desires access to the patient's records at multiple sites, the clinician submits a request to the patient, and the patient accesses the PDDS to retrieve the metadata related to the request. In some cases, the patient may grant access to the entirety of the records associated with the patient, and will correspondingly provide all of the patient's metadata to the clinician. The 'request' by the clinician may be implicit, wherein, for example, before a scheduled visit with the clinician, the patient may anticipate that the clinician will request the patient's metadata, and accesses the PDDS to retrieve the metadata before the visit.

In some cases, as noted above, the patient will grant access to only a selected set of the patient's records. In such cases, the patient will only provide the metadata related to a selection criteria, such as a span of dates, an anatomical region, etc. The selection criteria may also include exception criteria, wherein the user identifies a class of records that should not be provided to the clinician. Not illustrated in FIG. 1, the PDDS, or the patient's device, will include a filtering system wherein the patient provides the selection criteria to the filtering system, and the filtering system provides only the metadata that satisfies the selection criteria to the patient, for subsequent communication to the requesting clinician.

In some embodiments, the PDDS may be configured to store pre-defined selection criteria for some or all of the clinicians that have access to the patient's records and will filter any further data access requests based on these pre-defined selection criteria, unless specifically directed otherwise by the patient.

Upon receipt of the requested metadata from the patient, the clinician accesses the patient's records at each site identified in the received metadata. The clinician provides the (preferably encrypted) site-specific pseudo-ID (SPID) for the patient, as well as any further access information in each metadata entry to the identified site in the metadata. Given the access information, each site will decrypt the pseudo-ID and provide the clinical records of the identified patient to the clinician, but only if the access information is proper and the records at the specified location are associated with the pseudo-ID of the patient. One of skill in the art will recognize that if the metadata indicates that the records are stored at the site associated with the clinician, a different protocol may be used to access the locally stored clinical records.

Typically, the clinician will review the received clinical records, examine the patient as deemed necessary, then create a new clinical record that is stored at the clinician's associated site. Metadata related to this new clinical record is created and the clinician provides this metadata to the patient with a record-update request. If the patient approves this request, the patient provides this metadata to the **PDDS** for storage as the next entry in the patient's metadata records. To further protect access to the patient's clinical data, the patient may encrypt some or all of the metadata, particularly the site location and the pseudo-ID in the metadata that enables access to the data at the medical site. In some embodiments, as detailed further below, the PDDS may be configured to receive or retrieve the metadata directly from/to the clinician, upon approval by the patient.

As noted above, embodiments of this invention include security measures for protecting the security of the patient's records. A fundamental security criteria assures that only qualified/certified clinicians have access to the patient's clinical records stored at the multiple sites.

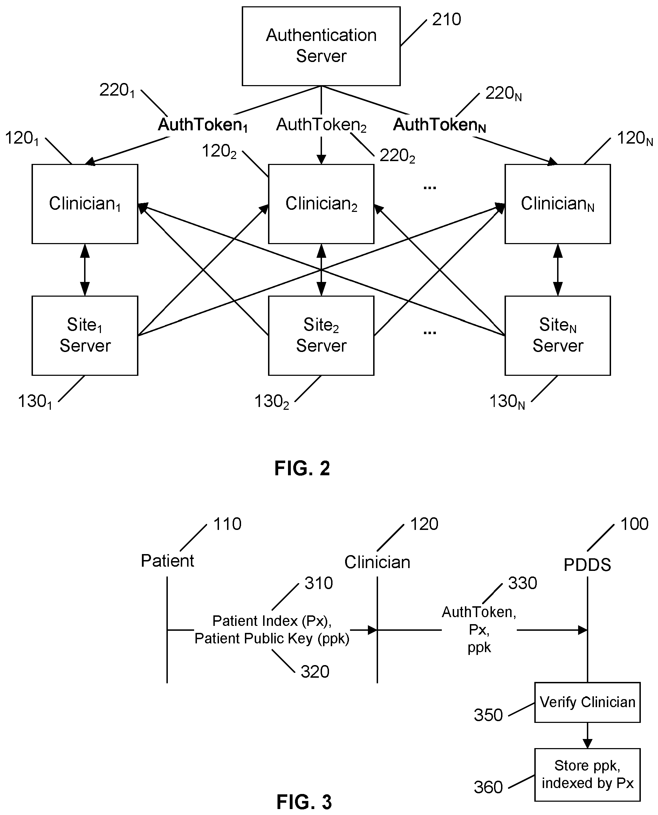

FIG. 2 illustrates an example clinician authentication system. In this embodiment, each clinician 120 authenticates himself/herself to an Authentication Service Provider. The actual implementation of this embodiment could be in multiple ways, including for example, an authentication server installed on a private cloud or one of the healthcare sites, which serves only for clinicians from a set of federated sites. In other embodiments, the Authentication Service Provider could be a well-known IdP (Identity Provider), such as Google, Microsoft, etc., which provides the functionality of federated authentication. Similarly the Authentication Service Provider could be a DID/SSI (Decentralized Identity/Self Sovereign Identity) that relies on some distributed storage or ledger to provide such an identity and authentication service.

In the example of FIG. 2, during the authentication process, a clinician 120ᵢ sends an authentication request to the Authentication Server 210 and, if the authentication is successful, receives a corresponding authentication token AuthTokenᵢ. The AuthToken will typically contain an identification of the clinician, signed by the Authentication Service Provider, and a cryptographic key, such as a public key of a public-private key pair assigned to the clinician. The AuthToken may also contain ancillary information, such as the site associated with the clinician, the clinician's specialty, etc. Later, when the clinician needs to access the PDDS or other sites, the clinician sends the AuthToken to the site, and proves his/her identity using a conventional cryptographic exchange based on the information contained in the AuthToken.

Preferably, access to the patient's clinical data is controlled by the clinical sites. In such embodiments, the Authentication Server authenticates the identity of the clinician, and the clinical sites have a list of clinicians authorized to access their records (or authorized to access a subset of their records). With the AuthToken, the clinician requests access to the patient's data at the clinical site, and the clinical site verifies the identity of the clinician based on the AuthToken. Upon successful verification, the clinical site determines whether this authenticated clinician is on their list of authorized clinicians for the requested records. The "list" of authorized clinicians may be an itemized list of each authorized clinician, or it may be in a more generalized form, such as "all authenticated clinicians at sites A, B, or C", or "all authenticated clinicians having specialty X". In some embodiments, the AuthToken may also serve as both an authentication of identity and an access authorization, wherein the list of authorized clinicians is in the form of "all authenticated clinicians".

In embodiments of this invention, "Open Authorization 2.0" (OAuth2) may be used to provide the AuthToken to the clinician. In OAuth2, the token is termed "Access Token". The clinician requests the Access Token from an "Authorization Server", and submits his/her credentials. The Authorization Server submits the clinician's credentials to a "Resource Owner"; in this case, the Resource Owner is an organization to which each of the medical sites ("Resource Servers") and the PDDS belong. The Resource Owner validates the clinician's credentials, and notifies the Authorization Server whether the clinician is authorized to obtain data stored at the Resource Servers. If authorized, the Authorization Server sends the Access Token to the clinician, which serves as the AuthToken of this invention.

One of skill in the art will recognize that the term "authentication token" is used herein in the generic sense, comprising any form of identification information that enables a clinician to prove his/her identity.

FIG. 3 illustrates an example message exchange for initiating a patient's access to the PDDS. This process is performed when the patient first visits a participating clinician. Any of a variety of access techniques may be used by the PDDS, such as having the patient choose a "user ID" and "password", or other conventional cryptographic verification techniques.

In the example embodiment represented in FIG. 3, the patient is in possession of a public-private key pair: a "ppk" - the patient's public key, and a "psk" - the patient's secret (private) key. These keys will be used to authenticate the patient each time the patient accesses the PDDS, typically via a "challenge"-"response" exchange between the PDDS and the patient, detailed further below. To facilitate access, the patient may choose a random number, such as a PIN, that the PDDS will use as an index (Patient Index, Px) to the multiple patients' records at the PDDS.

The patient 110 provides the Patient Index, Px 310, and the Patient's Public Key (ppk) 320 to the Clinician. The clinician communicates 330 the clinician's AuthToken, the Px, and the ppk to the PDDS 100. The PDDS 100 verifies 350 the clinician based on the AuthToken, and if the clinician is verified, the PDDS 100 creates an account for the patient, and stores 360 the ppk for this account, indexed by Px. Optionally, the patient may include an expiration date for the patient's public key, and this expiration date is communicated to the PDDS 100.

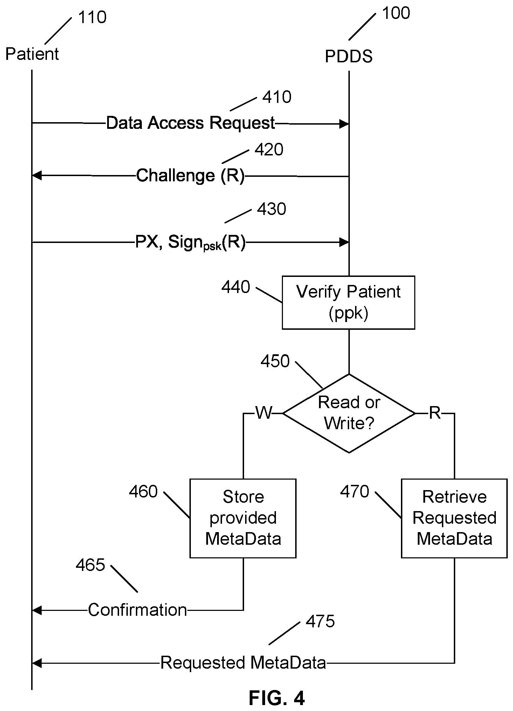

FIG. 4 illustrates an example flow diagram for providing access to the PDDS by the patient. Any number of techniques may be used to grant access to the patient, such as a log-in and password protocol, and others. In the example embodiment of FIG. 4, a conventional challenge-response protocol is used.

At 410, the patient provides a Data Access Request 410 to the PDDS 100. Typically, the Data Access Request 410 will be provided by the clinician to the patient, and will specify either the metadata that is to be written to the PDDS 100 (i.e. the metadata for a newly created clinical record at the clinician's site), or a request to read a set of metadata. As discussed above, the request may be filtered by the patient to provide only selected metadata to the clinician, such as metadata related to a particular anatomical region, a particular diagnosis, a particular time-frame, etc. This filtering may be performed at the patient's device, or the data access request 410 may include filtering criteria for the PDDS 100 to apply. Or, as noted above, the PDDS 100 may be configured with pre-defined filtering criteria for the clinician.

In response to the data access request 410, the PDDS 100 issues a challenge 420, typically in the form of a random number R. In response to the challenge, the patient signs the challenge using the patient's secret key (psk), and communicates 430 this signed challenge Signₚₛₖ(R), and the PDDS finds and verifies the patient based on the PDDS's storage of patients' public keys (ppk). The PDDS 100 could search through all of its patient accounts to find a patient public key (ppk) that corresponds to the patient having the patient secret key (psk). However, in this example embodiment, the patient communicates his/her patient index (Px), and the PDDS 100 only searches through the patient accounts having that patient index to find the patient having the patient public key that corresponds to the patient secret key that was used to sign the challenge (Signₚₛₖ(R)).

Upon finding/verifying the patient based on the signed challenge, the PDDS 100 determines whether the data access request 410 is for a reading of the stored metadata, wherein the clinician is a 'data requestor', or for a writing of a new set of metadata, wherein the clinician is a 'data provider' at 450. If the request is to write a new set of metadata, the PDDS 100 adds this new set of metadata provided by the clinician to the patient's account. As noted above, the patient may encrypt some or all of the metadata before submitting it to the PDDS 100 for storage.

If the data access request 410 is for a reading of the metadata, the PDDS 100 retrieves the metadata requested by the clinician, at 470, and communicates 475 the requested metadata to the patient. If the metadata was encrypted by the patient, the patient decrypts the metadata before sending it to the requesting clinician. As noted above, the requested metadata may be selected metadata based on filtering using the patient's selection criteria. Alternatively, the PDDS 100 could be configured to always return all of the patient's metadata to the patient, and the patient may subsequently apply the selection filtering before communicating the selected metadata to the clinician.

One of skill in the art will recognize that the PDDS 100 could be configured to interact with both the clinician and the patient, to avoid having the patient relay the metadata to and from the clinician. In this case, the clinician could initiate the data access request 410 while simultaneously notifying the patient of this request. If the patient concurs, the patient will respond with the challenge response of Px, Signₚₛₖ(R) 430; otherwise, in the absence of a proper challenge response, the PDDS 100 will notify the clinician that the request 410 has not been approved by the patient. In addition to the challenge response 430, the patient 110 may also provide selection filtering criteria to the PDDS 100 for each data access request 410.

The PDDS 100 may also be configured to send the Confirmation 465 and/or the requested metadata 475 directly to the clinician, unless directed otherwise by the patient.

As noted above, the metadata provided to the PDDS 100 may be encrypted by the patient, preferably using a different key than the patient's key(s) that is used to access the PDDS 100.

In some embodiments, some or all of the metadata, and particularly the site location (SURL) and the site's pseudo-ID for the patient (SPID), may be encrypted using a conventional symmetric key to reduce computation overhead for encryption and decryption.

In other embodiments, particularly if the PDDS is configured to transmit the metadata directly to the clinician, a conventional Proxy Re-Encryption (PRE) technique may be used. Proxy re-encryption allows a first message encrypted by a first user using a first public key, to be transformed by a proxy device into a second message that a second user can decrypt, without the proxy device having access to the plain-text (unencrypted) message.

In this case, the patient acquires a public-private key pair (skₚ, pkₚ) and the clinician acquires a public-private key pair (sk_{c}, pk_{c}). During the writing process, the patient encrypts the metadata using the patient's public key (pkₚ) and stores the encrypted metadata E_{PKp}(M) on the PDDS.

When the clinician requests the metadata, the clinician sends his/her public key pk_{c} to the patient. If the patient approves the request, the patient uses a proxy re-encryption scheme to create a re-encryption key based on the patient's private key skₚ and the clinician's public key pk_{c}. The patient sends this re-encryption key to the PDDS with the data access request. The PDDS retrieves the requested patient-encrypted metadata and uses the re-encryption key to transform the patient-encrypted metadata into re-encrypted metadata that can be decrypted by the clinician's private key sk_{c}. The PDDS sends this re-encrypted metadata to the clinician, who then decrypts the metadata using the clinician's private key sk_{c}.

Preferably, the proxy re-encryption scheme used to provide the re-encryption key is configured to generate a unique re-encryption key for each requested data access, using for example a random number selected by the patient for each request. In this case, the proxy re-encryption scheme may require the patient to communicate ancillary data with the re-encryption key to the PDDS.

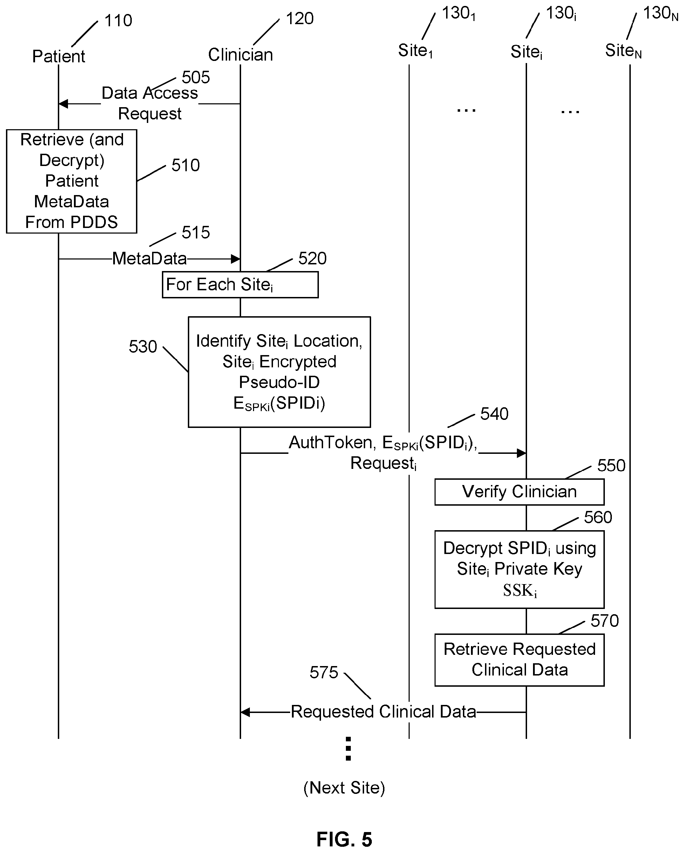

FIG. 5 illustrates an example flow diagram for providing the patient's clinical data to the clinician, based on the patient's metadata from the PDDS.

The clinician 120 initiates the process by sending a data access request to the patient 110. The patient 110 retrieves the requested metadata from the PDDS using the example process of FIG. 4, and sends the metadata to the clinician 120. If the metadata is encrypted, the patient 110 decrypts it and sends the decrypted metadata to the clinician 120. As indicated above, the PDDS may be configured to communicate the metadata to the clinician 120 directly, after the patient 110 approves the requested access by the clinician. Typically, the patient's metadata will include multiple entries, each entry corresponding to clinical data stored at a particular site. The clinician iteratively processes 520 the metadata for each site.

FIG. 5 illustrates the processing of the metadata for a siteᵢ; the metadata for each site is processed similarly. However, at the site that is associated with the requesting clinician, a different protocol may be used to access the locally stored clinical data.

At 530, the clinician 120 processes the metadata to identify the Site Location; (Siteᵢ URL, or SURLᵢ) and the Pseudo-ID; (SPIDᵢ) for the patient 110 at Siteᵢ 130ᵢ. Preferably, the Pseudo-ID; is encrypted by the siteᵢ using a public key (SPKᵢ) of the siteᵢ when the metadata is first created by the siteᵢ and subsequently stored at the PDDS by the patient. The metadata may also include an identification of the clinical data to be retrieved, such as a record pointer or other identifying information.

The clinician 120 sends 540 his/her AuthToken, the encrypted SPID_{i,} (E_{SPKi}(SPIDᵢ)), and the data request to the identified Siteᵢ. Some sites may have multiple public keys; if so, the metadata will include an identification (e.g. index, or Key ID) of which public key was used when the site encrypted the SPID. The data request includes the metadata information that facilitates retrieval of the patient's clinical data, as well as other information.

At 550, the Siteᵢ 130ᵢ verifies the clinician 120 based on the AuthToken, and upon successful verification, decrypts 560 the SPIDᵢ using the Site's private/secret key SSKᵢ. Using the decrypted SPIDᵢ, and other metadata information in the request 540, the Siteᵢ 130ᵢ retrieves 570 the clinical data of the patient 110, and sends it to the clinician 120 (575).

In embodiments of this invention, the PDDS enables replacement of the key(s) used to verify the patient, as well as replacement of some or all of the stored metadata. For example, a medical site may wish to re-encrypt the encrypted SPID in the stored metadata. This re-encryption may be based on a schedule of key updates for enhanced security. The re-encryption may also be based on discovery of a possible compromise of prior keys, or other security concerns. In like manner, the patient may wish to re-encrypt the patient-encrypted stored metadata for any of a variety of reasons.

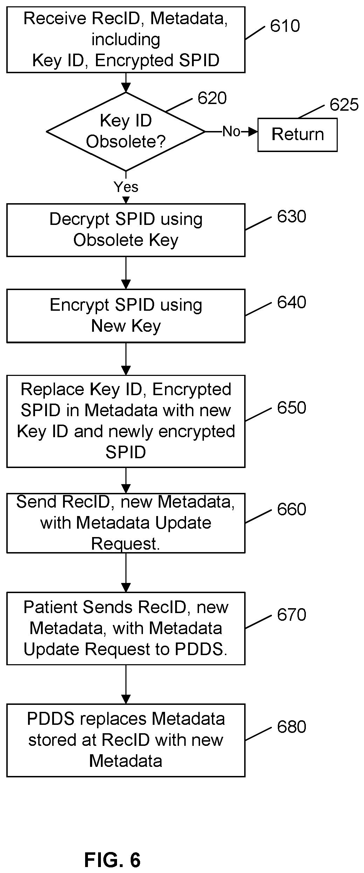

FIG. 6 illustrates an example flow diagram for re-encrypting the site-encrypted pseudo-ID in the stored metadata at the PDDS. The site has multiple keys, each identified by a Key ID (index to keys). In this embodiment, each time a data access request is received at the site, the site determines whether the key is 'obsolete' and should be replaced. This process may be performed before or after the data access request is serviced.

At 610, the site receives the metadata associated with the data request, with an identification of which record (RecID) in the PDDS this metadata is located. This RecID enables the patient to identify at which record the metadata should be replaced by the re-encrypted metadata. Typically, the data request will come from a clinician, but in some embodiments, the site may allow a patient to directly request the clinical records associated with the patient, or at least directly request that the metadata be checked for obsolete keys. Alternatively, the site may initiate this process by requesting receipt of some or all of its metadata stored at the PDDS; this request may be sent to the clinician or the patient.

At 620, the site determines whether the key that was used to encrypt the pseudo-ID is obsolete, based on the Key ID. As noted above, the keys may be determined to be obsolete based on a schedule of replacement keys, or based on the occurrence of particular events. If the key is not obsolete, no action is taken 625.

If the key is obsolete, the site decrypts the SPID using the original, obsolete key at 630, and re-encrypts it using a new key, which is identified by a new Key ID, at 640.

At 650, the site replaces the Key ID and encrypted SPID with the new Key ID and newly encrypted SPID in the metadata. This replacement metadata is communicated to the patient with a Metadata Update Request with an identification of the record RecID at which the metadata is to be replaced, at 660. The site may send the replacement metadata and update request to the clinician, for forwarding to the patient.

At 670, the patient (or site) sends the replacement metadata and update request to the PDDS, and the PDDS replaces the stored metadata at the record identified by RecID with this replacement metadata, at 680.

One of skill in the art will recognize that a similar process may be used to re-encrypt other data. For example, if the stored metadata is encrypted by the patient using a public/private key pair, and the patient wants to re-encrypt the metadata with a new public/private key pair, the patient can send the new public key to the PDDS and request each encrypted metadata record from the PDDS. The patient will decrypt the encrypted metadata using the patient's original private key, re-encrypt it using the patient's new public key, and return the newly encrypted metadata with a metadata update request to the PDDS.

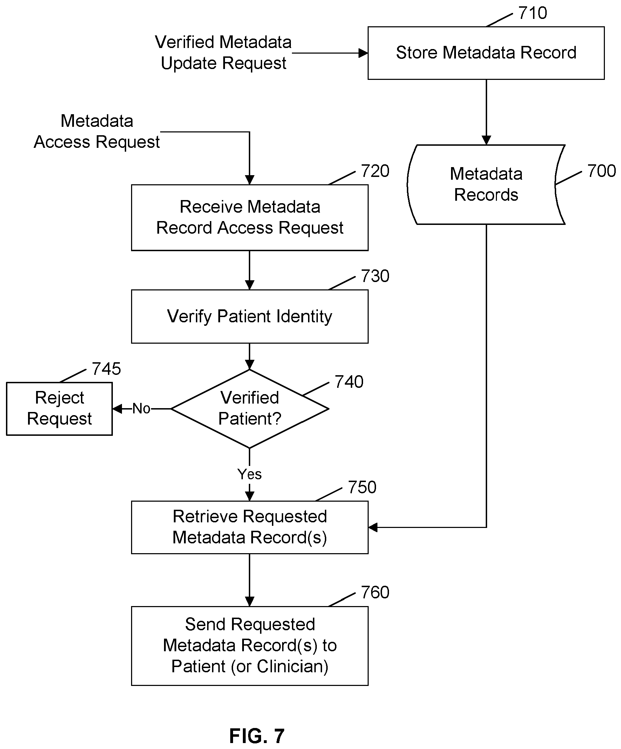

FIG. 7 illustrates a flow diagram of an example method of storing and accessing metadata records of a patient at a PDDS.

At 710, the method comprises storing 710 metadata records 700 of a patient at the PDDS. As detailed above, appropriate verifications are performed to verify that the metadata is provided by the patient.

As also detailed above, the metadata is associated with the clinical records of the patient at a plurality of clinical sites. The metadata is provided by the clinical site for each new clinical record stored at the site, and includes an identification of the clinical site and a site-specific pseudo-ID associated with the patient at the clinical site. The pseudo-ID is necessary to access the clinical data at the clinical site, and is preferably encrypted by the clinical site. The metadata may also include other information that facilitates selective filtering of the records by the patient before sending the metadata to a particular data requestor.

Subsequent to the storing of the metadata records 700, the PDDS may receive 720 a metadata record access request from the patient. This access request may include a request for one or more metadata records. The patient's identity is verified 730, as detailed above, and if verified 740, the requested metadata record(s) is retrieved 750 from the stored metadata records 700.

At 760, the requested metadata records are sent to the patient, for forwarding to the requesting clinician, or, as detailed above, sent directly to the clinician if authorized by the patient. Not illustrated in FIG. 7, the patient may selectively limit/filter the metadata that is communicated to the requesting clinician. This filtering may be performed at the PDDS, or at the patient's device. If performed at the PDDS, the patient may include the filtering criteria in the metadata access request.

In a preferred embodiment, a data access tracking system is provided to record interaction messages among patients, clinicians, sites, and the PDDS as access records. Each access record preferably includes information associated with each interaction message and a timestamp. The interaction messages may include, for example, a registration request, a record access request, a record update request, a clinical data access request, a request approval, and a request denial.

The data access tracking system may be an independent system that receives the interaction messages from the various components of the distributed clinical data management system of this invention, or it also may be distributed among the various components. For example, the Authentication Server 210 may maintain a log that identifies each clinician, the date of issuance of the AuthToken, and other information such as the site associated with the clinician.

The PDDS 100 may maintain a log related to the patient, a log related to the clinician, and a log related to metadata access. The patient log may contain an identification of each patient, a record of changes to each patient's index Px, and other changes. The clinician log may contain information extracted from the AuthToken and information that uniquely identifies each clinician. The metadata access log may contain information related to each access to the patient's metadata, including an identification of the patient and requesting clinician, a date-time stamp, and an identification of the metadata that was read or written. In like manner, each Site 130 may maintain a log that identifies the patient, the clinician, a date-time stamp, and an identification of which clinical data was read or written. In such a distributed embodiment, the data access tracking system includes a management system that collects and consolidates the information (interaction messages) from the various logs.

The data access tracking system includes a user interface that enables review of the access records. The review of the access records may be selectively limited. For example, the patient may have access to all interaction messages related to the patient or the patient's records, whereas the clinician may only have access to interaction messages in which the clinician was a party.

While the invention has been illustrated and described in detail in the drawings and foregoing description, such illustration and description are to be considered illustrative or exemplary and not restrictive.

Of particular note, although the invention is presented as comprising a distinct PDDS 100 that is separate from the patient's device 110 and the clinical sites 130, one of skill in the art will recognize that the PDDS 100 may be a centralized service on a cloud server, or within a server at a selected clinical site 130. In like manner, the PDDS can be a privately managed application that is embodied in the patient device 110. That is, for example, each patient may be provided a PDDS "app" for storing his/her individual metadata records. This PDDS app may be embodied as an application on the patient's personal computer, or, for example, as a "digital wallet" on the patient's mobile device. An advantage of providing an individualized PDDS to each patient is that such an integration will facilitate automation of tasks such as receiving, decrypting, filtering, and forwarding the metadata to the clinician. Such an embodiment also reduces the risks associated with a data breach at a centralized PDDS.

Additionally, although the invention is presented in the context of having the patient involved in each metadata access request, it is possible to operate the invention in an embodiment wherein the patient authorizes one or more clinicians to access the PDDS 100 at any time. In this case, the patient's approval is only required for creating this authorization, or for data access requests that have not been pre-approved. In such an embodiment, the clinician may contact the PDDS 100 directly, and if the clinician has been pre-approved, the PDDS 100 provides the metadata directly to the clinician.

In general, each entity described in this specification may be embodied as, or in, a device or apparatus. The device or apparatus may comprise one or more (micro)processors which execute appropriate software. The processor(s) of a respective entity may be embodied by one or more of these (micro)processors. Software implementing the functionality of a respective entity may have been downloaded and/or stored in a corresponding memory or memories, e.g., in volatile memory such as RAM or in non-volatile memory such as Flash. Any input and/or output interfaces may be implemented by respective interfaces of the device or apparatus. In general, each functional unit of a respective entity may be implemented in the form of a circuit or circuitry. A respective entity may also be implemented in a distributed manner, e.g., involving different devices or apparatus, or in a client-server configuration.

For example, the patient data directory system may be embodied by a computer such as a workstation, a server, or a distributed system of servers, e.g., a cloud-based system. Another example is that the patient device may be a mobile device such as a smartphone, tablet, smart glasses, etc. or a computer. Another example is that each of the data provider device and the data requestor device may be a mobile device, a personal computer, a workstation, etc. In some cases, a respective device may be implemented in a client-server configuration in which a client device is provided, e.g., with a user interface and a display, and in which a remote server is configured to provide the functionality described in relation to the respective device via client-server communication. For example, the functionality described for the patient device, the data provider device, and/or the data requestor device may each be provided by a webservice which may be accessed from a mobile device or computer.

## Claims

1. A patient data directory system (PDDS) (100) comprising:
a plurality of records associated with a patient (110),
patient verification information (160) which uses one or more keys to represent the patient in the PDDS and to link each of the patient's plurality of records, and
an access control system that controls access to the plurality of records,
wherein the access to the plurality of records is controlled by the patient,
wherein the patient can approve or deny access to clinical data of the patient by each of a plurality of data requestors (120), wherein each of the plurality of data requestors is provided with an authentication token,
wherein the plurality of records comprises metadata that is associated with clinical data of the patient that is stored at a plurality of clinical sites (130),
wherein each clinical site identifies the patient using a site-specific pseudo-ID (SPID) of the patient,
wherein the metadata (180) of each record identifies a location (182) of the patient's clinical data at a corresponding clinical site, and the SPID (184) of the patient at the corresponding clinical site,
wherein the access control system comprises an identity verification process to verify that a user of the system is the patient that controls access to the metadata records, and
wherein if the user is verified as being the patient, and data access is approved for the data requestor, the metadata of all or a selected set of records is communicated from the PDDS via the patient to the data requestor to enable the data requestor to access the clinical data of the patient associated with said records at the corresponding clinical site based on the SPID of the patient at the corresponding clinical site and by providing the authentication token to the corresponding clinical site.

2. The PDDS (100) of claim 1,
wherein one or multiple keys are used to represent the patient in the PDDS and to link each of the patient's plurality of records,
wherein the one or multiple keys comprise one or multiple random keys provided by the patient and stored in PDDS,
wherein the one or more multiple keys do not reveal identification information of the patient, and
wherein the verification mechanism uses a cryptographic protocol based on the one or more multiple keys.

3. The PDDS (100) of claim 2, wherein the PDDS enables the patient, after passing identity verification, to change the one or multiple keys that are used to represent the patient in the PDDS and to link each of the patient's plurality of records.

4. The PDDS (100) of claim 1,
wherein the verification mechanism comprises a cryptographic protocol that requires a one-time setup and subsequent challenge-response authentications,
wherein the one-time setup requires the patient to provide a public key and a private key of a public/private key pair, and
wherein the private key is stored by the patient and the public key is stored in the PDDS via a data provider who registers a new SPID to the PDDS for the patient.

5. The PDDS (100) of claim 1,
wherein the data provider provides the authentication token to the PDDS to register a new SPID for the patient at the PDDS.

6. The PDDS (100) of claim 1,
wherein a data provider provides the metadata associated with the clinical data of the patient,
wherein the data provider sends a record-update request including the metadata to the patient,
wherein the patient approves or denies the request, and
wherein if the request is approved, the patient encrypts the metadata to create patient-encrypted metadata that is stored in the PDDS.

7. The PDDS (100) of claim 1,
wherein the metadata is encrypted by the patient to form patient-encrypted metadata that is stored at the PDDS, and
wherein the PDDS comprises a proxy re-encryption system that transforms the patient-encrypted metadata into re-encrypted metadata that can be decrypted by the data requestor.

8. The PDDS (100) of claim 1,
wherein the metadata is encrypted by the patient to form patient-encrypted metadata that is stored at the PDDS, and
wherein the PDDS sends the patient-encrypted metadata to the patient for subsequent communication of the metadata to the data requestor.

9. The PDDS (100) of claim 1,
wherein the SPID in the metadata that is communicated to the data requestor is encrypted using a cryptographic key owned by the corresponding clinical site, and
wherein the data requestor sends the encrypted SPID to the corresponding clinical site, which can then be decrypted as needed by the clinical site.

10. The PDDS (100) of claim 9,
wherein a clinical site possesses multiple cryptographic keys, each identified by a unique key ID,
wherein the metadata includes the key ID and the SPID encrypted using the key referenced by the key ID, and
wherein the key ID and the encrypted SPID are communicated to the data requester to identify the key used for encryption by the clinical site.

11. The PDDS (100) of claim 10, wherein the PDDS enables replacement of the key ID and the SPID that is encrypted using the key referenced by the key ID with a new key ID and the SPID that is encrypted using the new key ID.

12. The PDDS (100) of claim 1, wherein the PDDS includes a plurality of metadata records for a plurality of patients.

13. The PDDS (100) of claim 1,
wherein the patient provides selective access to the metadata to each data requestor, and wherein the selective access is based on ancillary information in the metadata,
wherein the ancillary information comprises one or more of:
an anatomical region associated with the clinical data,
a time-frame related to the clinical data, and
a diagnosis associated with the clinical data.

14. The PDDS (100) of claim 1,
wherein a public-private key pair is associated with the patient,
wherein the public key of the public-private key pair is communicated to the PDDS, and
wherein the PDDS system enables the patient, after passing identity verification, to change the public key in the PDDS to a new public key corresponding to a new public-private key pair of the patient.

15. A patient device for use by a patient (110), comprising:
a communication system for communicating with a data requestor (120) and a patient data directory system (PDDS) (100), and
a user interface;
wherein the PDDS is configured to store records related to clinical data of the patient stored at a plurality of clinical sites (130) and patient verification information which uses one or more keys to represent the patient in the PDDS and to link each of the patient's plurality of records,
wherein the records comprise metadata associated with the clinical data at each of the plurality of clinical sites,
wherein the metadata (180) includes an identification of a location (182) of the clinical data at each of the plurality of clinical sites, and a site-specific pseudo-ID (SPID) (184) of the patient,
wherein, when the patient device receives a record-access request for all or a selected set of records from the data requestor:
the patient device, via the user interface, presents the request, and receives an approval or denial response from the patient,
if the request is approved:
the patient device uses the cryptographic system to gain access to the metadata records of the patient at the PDDS,
the patient device uses the communication system to communicate the record-access request to the PDDS,
the patient device uses the communication system to receive, in response to the record-access request, the metadata records of the patient from the PDDS, and
the patient device uses the communication system to communicate the metadata selected by the patient to the data requestor, to facilitate access to the clinical data of the patient associated with all or the selected set of records stored at the plurality of clinical sites by the data requestor.

16. The patient device of claim 15, comprising a cryptographic system,
wherein, when the patient device receives a record-update request from the data provider:
the patient device, via the user interface, presents the request to the patient, and receives an approval or denial response from the patient,
the patient device uses the cryptographic system to gain access to the PDDS, and
the patient device uses the communication system to communicate the metadata to the PDDS.

17. The patient device of claim 15, wherein the patient device includes the PDDS (100).

18. A data provider device for use by a data provider, comprising:
a communication system for communicating with a patient (110) and a clinical site server associated with the data provider;
wherein a patient data directory system (PDDS) (100) is configured to store records related to clinical data of the patient stored at a plurality of clinical sites (130),
wherein the records comprise metadata associated with the clinical data at each of the plurality of clinical sites,
wherein the metadata (180) includes an identification of a location (182) of the clinical data at each of the plurality of clinical sites, and a site-specific pseudo-ID (SPID) (184) of the patient,
wherein the data provider device uses the communication system to store the patient's clinical data in a clinical site server and receive corresponding metadata in response, and
wherein the data provider device uses the communication system to communicate a record-update request including the metadata to the patient for subsequent submission to the PDDS, and if the patient is a new patient, communicate patient verification information to the PPDS to represent the patient in the PDDS and to link each of the patient's records, wherein the patient verification information uses one or more keys to represent the patient in the PDDS.

19. A data requestor device comprising:
a communication system for communicating with a patient (110) and a plurality of clinical sites (130);
wherein the patient has access to a patient data directory system (PDDS) (100) that is configured to store records related to clinical data of the patient stored at the plurality of clinical sites and patient verification information which uses one or more keys to represent the patient in the PDDS and to link each of the patient's plurality of records,
wherein the records comprise metadata associated with the clinical data at each of the plurality of clinical sites,
wherein the metadata (180) includes an identification of a location (182) of the clinical data at each of the plurality of clinical sites, and a site-specific pseudo-ID (SPID) (184) of the patient,
wherein the data requestor device is provided with an authentication token,
wherein the data requestor device uses the communication system to communicate a data access request for all or a selected set of records to the patient,
wherein the data requestor device uses the communication system to receive the metadata associated with the clinical data from the patient, and
wherein the data requestor device uses the communication system to access the clinical data of the patient associated with all or the selected set of records stored at the plurality of clinical sites based on the metadata and by providing the authentication token to a corresponding clinical site.

20. A computer implemented method (700) of managing access to clinical records of a patient, comprising:
storing (710) a plurality of records that facilitate access to the clinical records of the patient,
wherein the plurality of records comprises metadata that is associated with clinical data of the patient that is stored at a plurality of clinical sites,
wherein the metadata of each record comprises an identification of a clinical site of the plurality of clinical sites and a site-specific pseudo-ID (SPID) associated with the patient at the clinical site,
wherein the site-specific pseudo-ID is necessary to access the clinical data at the clinical site,
receiving (720) a record access request from a user,
verifying (730) that the user is the patient that controls access to the metadata records,
if the user is verified (740) to be the patient and access to the clinical data of the patient is approved for a data requestor by the patient, providing (750, 760) the metadata requested in the record access request to the patient; and
communicating the metadata from the patient to the data requestor to enable the data requestor to access the clinical data of the patient at the corresponding clinical site based on the SPID of the patient at the corresponding clinical site.

21. A non-transitory computer-readable medium comprising a program that, when executed by a processing system, causes the processing system to perform the method of claim 20.

## Patentansprüche

1. Patientendatenverzeichnissystem (Patient Data Directory System, PDDS) (100), umfassend:
eine Mehrzahl von Datensätzen, die einem Patienten (110) zugeordnet sind,
Patientenverifizierungsinformationen (160), die einen oder mehrere Schlüssel verwenden, um den Patienten im PDDS darzustellen und jeden der Mehrzahl von Datensätzen des Patienten zu verknüpfen, und
ein Zugriffssteuerungssystem, das den Zugriff auf die Mehrzahl von Datensätzen steuert,
wobei der Zugriff auf die Mehrzahl von Datensätzen durch den Patienten kontrolliert wird,
wobei der Patient den Zugriff auf klinische Daten des Patienten durch jeden einer Mehrzahl von Datenanforderern (120) genehmigen oder verweigern kann, wobei jeder der Mehrzahl von Datenanforderern mit einem Authentifizierungstoken versehen ist,
wobei die Mehrzahl von Datensätzen Metadaten umfasst, die klinischen Daten des Patienten zugeordnet sind, die bei einer Mehrzahl von klinischen Einrichtungen (130) gespeichert sind,
wobei jede klinische Einrichtung den Patienten unter Verwendung einer einrichtungsspezifischen Pseudo-ID (Site-Specific Pseudo-ID, SPID) des Patienten identifiziert,
wobei die Metadaten (180) jedes Datensatzes einen Ort (182) der klinischen Daten des Patienten bei einer entsprechenden klinischen Einrichtung und die SPID (184) des Patienten bei der entsprechenden klinischen Einrichtung identifizieren,
wobei das Zugriffssteuerungssystem einen Identitätsverifizierungsprozess umfasst, um zu verifizieren, dass ein Benutzer des Systems der Patient ist, der den Zugriff auf die Metadatendatensätze kontrolliert, und
wobei, wenn der Benutzer als der Patient verifiziert wird und der Datenzugriff für den Datenanforderer genehmigt wird, die Metadaten aller oder eines ausgewählten Satzes von Datensätzen vom PDDS über den Patienten an den Datenanforderer übermittelt werden, um dem Datenanforderer zu ermöglichen, auf die klinischen Daten des Patienten, die den Datensätzen zugeordnet sind, bei der entsprechenden klinischen Einrichtung basierend auf der SPID des Patienten bei der entsprechenden klinischen Einrichtung und durch Bereitstellen des Authentifizierungstokens an die entsprechende klinische Einrichtung zuzugreifen.

2. PDDS (100) nach Anspruch 1,
wobei ein oder mehrere Schlüssel verwendet werden, um den Patienten im PDDS darzustellen und jeden der Mehrzahl von Datensätzen des Patienten zu verknüpfen,
wobei der eine oder die mehreren Schlüssel einen oder mehrere Zufallsschlüssel umfassen, die durch den Patienten bereitgestellt und in PDDS gespeichert werden,
wobei der eine oder die mehreren Schlüssel keine Identifikationsinformationen des Patienten offenbaren, und
wobei das Verifizierungsmittel ein kryptografisches Protokoll basierend auf dem einen oder den mehreren Schlüsseln verwendet.

3. PDDS (100) nach Anspruch 2, wobei das PDDS es dem Patienten ermöglicht, nach Bestehen einer
Identitätsverifizierung den einen oder die mehreren Schlüssel zu ändern, die verwendet werden, um den Patienten im PDDS darzustellen und jeden der Mehrzahl von Datensätzen des Patienten zu verknüpfen.

4. PDDS (100) nach Anspruch 1,
wobei das Verifizierungsmittel ein kryptografisches Protokoll umfasst, das eine einmalige Initialisierung und nachfolgende Aufforderung-Antwort-Authentifizierungen erfordert,
wobei die einmalige Initialisierung erfordert, dass der Patient einen öffentlichen Schlüssel und einen privaten Schlüssel eines Paars von öffentlichen/privaten Schlüsseln bereitstellt, und
wobei der private Schlüssel durch den Patienten gespeichert wird und der öffentliche Schlüssel im PDDS über einen Datenanbieter gespeichert wird, der eine neue SPID beim PDDS für den Patienten registriert.

5. PDDS (100) nach Anspruch 1,
wobei der Datenanbieter das Authentifizierungstoken dem PDDS bereitstellt, um eine neue SPID für den Patienten beim PDDS zu registrieren.

6. PDDS (100) nach Anspruch 1,
wobei ein Datenanbieter die Metadaten bereitstellt, die den klinischen Daten des Patienten zugeordnet sind,
wobei der Datenanbieter eine Datensatzaktualisierungsanforderung einschließlich der Metadaten an den Patienten sendet,
wobei der Patient die Anforderung genehmigt oder ablehnt, und
wobei, wenn die Anforderung genehmigt wird, der Patient die Metadaten verschlüsselt, um patientenverschlüsselte Metadaten zu erzeugen, die im PDDS gespeichert sind.

7. PDDS (100) nach Anspruch 1,
wobei die Metadaten durch den Patienten verschlüsselt werden, um patientenverschlüsselte Metadaten zu bilden, die
im PDDS gespeichert werden, und
wobei das PDDS ein Proxy-Neuverschlüsselungssystem umfasst, das die patientenverschlüsselten Metadaten in neu verschlüsselte Metadaten transformiert, die durch den Datenanforderer entschlüsselt werden können.

8. PDDS (100) nach Anspruch 1,
wobei die Metadaten durch den Patienten verschlüsselt werden, um patientenverschlüsselte Metadaten zu bilden, die
im PDDS gespeichert werden, und
wobei das PDDS die patientenverschlüsselten Metadaten an den Patienten zur nachfolgenden Übermittlung der Metadaten an den Datenanforderer sendet.

9. PDDS (100) nach Anspruch 1,
wobei die SPID in den Metadaten, die an den Datenanforderer übermittelt werden, unter Verwendung eines kryptografischen Schlüssels verschlüsselt wird, der der entsprechenden klinischen Einrichtung gehört, und
wobei der Datenanforderer die verschlüsselte SPID an die entsprechende klinische Einrichtung sendet, die dann nach Bedarf durch die klinische Einrichtung entschlüsselt werden kann.

10. PDDS (100) nach Anspruch 9,
wobei eine klinische Einrichtung mehrere kryptografische Schlüssel besitzt, die jeweils durch eine eindeutige Schlüssel-ID identifiziert sind,
wobei die Metadaten die Schlüssel-ID und die SPID beinhalten, die unter Verwendung des durch die Schlüssel-ID referenzierten Schlüssels verschlüsselt ist, und
wobei die Schlüssel-ID und die verschlüsselte SPID an den Datenanforderer übermittelt werden, um den Schlüssel zu identifizieren, der zur Verschlüsselung durch die klinische Einrichtung verwendet wird.

11. PDDS (100) nach Anspruch 10, wobei das PDDS ein Ersetzen der Schlüssel-ID und
der SPID, die unter Verwendung des durch die Schlüssel-ID referenzierten Schlüssels verschlüsselt ist, durch eine neue Schlüssel-ID und die SPID, die unter Verwendung der neuen Schlüssel-ID verschlüsselt ist, ermöglicht.

12. PDDS (100) nach Anspruch 1, wobei das PDDS eine Mehrzahl von Metadatendatensätzen
für eine Mehrzahl von Patienten beinhaltet.

13. PDDS (100) nach Anspruch 1,
wobei der Patient selektiven Zugriff auf die Metadaten für jeden Datenanforderer bereitstellt, und
wobei der selektive Zugriff auf Zusatzinformationen in den Metadaten basiert,
wobei die Zusatzinformationen eines oder mehrere von Folgendem umfassen:
eine anatomische Region, die den klinischen Daten zugeordnet ist,
einen Zeitrahmen in Bezug auf die klinischen Daten, und
eine Diagnose, die den klinischen Daten zugeordnet ist.

14. PDDS (100) nach Anspruch 1,
wobei dem Patienten ein öffentlich-privates Schlüsselpaar zugeordnet ist,
wobei der öffentliche Schlüssel des öffentlich-privaten Schlüsselpaars an das PDDS übermittelt wird, und
wobei das PDDS-System es dem Patienten ermöglicht, nach Bestehen einer Identitätsverifizierung den öffentlichen Schlüssel im PDDS in einen neuen öffentlichen Schlüssel zu ändern, der einem neuen öffentlich-privaten Schlüsselpaar des Patienten entspricht.

15. Patientenvorrichtung zur Verwendung durch einen Patienten (110), umfassend:
ein Kommunikationssystem zum Kommunizieren mit einem Datenanforderer (120) und einem Patientendatenverzeichnissystem (Patient Data Directory System, PDDS) (100), und
eine Benutzerschnittstelle;
wobei das PDDS dazu ausgelegt ist, Datensätze in Bezug auf klinische Daten des Patienten, die bei einer Mehrzahl von klinischen Einrichtungen (130) gespeichert sind, und Patientenverifizierungsinformationen, die einen oder mehrere Schlüssel verwenden, um den Patienten im PDDS darzustellen und jeden der Mehrzahl von Datensätzen des Patienten zu verknüpfen, zu speichern,
wobei die Datensätze Metadaten umfassen, die den klinischen Daten bei jeder der Mehrzahl von klinischen Einrichtungen zugeordnet sind,
wobei die Metadaten (180) eine Identifikation eines Orts (182) der klinischen Daten bei jeder der Mehrzahl von klinischen Einrichtungen und eine einrichtungsspezifische Pseudo-ID (Site-Specific Pseudo-ID, SPID) (184) des Patienten beinhalten, wobei, wenn die Patientenvorrichtung eine Datensatzzugriffsanforderung für alle oder einen ausgewählten Satz von Datensätzen von dem Datenanforderer empfängt:
die Patientenvorrichtung über die Benutzerschnittstelle die Anforderung vorlegt und eine Genehmigungs- oder Ablehnungsantwort von dem Patienten empfängt,
wenn die Anforderung genehmigt wird:
die Patientenvorrichtung das kryptografische System verwendet, um Zugriff auf die Metadatendatensätze des Patienten im PDDS zu erhalten,
die Patientenvorrichtung das Kommunikationssystem verwendet, um die Datensatzzugriffsanforderung an das PDDS zu übermitteln,
die Patientenvorrichtung das Kommunikationssystem verwendet, um als Reaktion auf die Datensatzzugriffsanforderung die Metadatendatensätze des Patienten vom PDDS zu empfangen, und
die Patientenvorrichtung das Kommunikationssystem verwendet, um die durch den Patienten ausgewählten Metadaten an den Datenanforderer zu übermitteln, um den Zugriff auf die klinischen Daten des Patienten, die allen oder dem ausgewählten Satz von Datensätzen zugeordnet sind, die bei der Mehrzahl von klinischen Einrichtungen gespeichert sind, durch den Datenanforderer zu erleichtern.

16. Patientenvorrichtung nach Anspruch 15, umfassend ein kryptografisches System,
wobei, wenn die Patientenvorrichtung eine Datensatzaktualisierungsanforderung von dem Datenanbieter empfängt:
die Patientenvorrichtung über die Benutzerschnittstelle dem Patienten die Anforderung vorlegt und eine Genehmigungs- oder Ablehnungsantwort von dem Patienten empfängt,
die Patientenvorrichtung das kryptografische System verwendet, um Zugriff auf das PDDS zu erhalten, und
die Patientenvorrichtung das Kommunikationssystem verwendet, um die Metadaten an das PDDS zu übermitteln.

17. Patientenvorrichtung nach Anspruch 15, wobei die Patientenvorrichtung das PDDS (100) beinhaltet.

18. Datenanbietervorrichtung zur Verwendung durch einen Datenanbieter, umfassend:
ein Kommunikationssystem zum Kommunizieren mit einem Patienten (110) und einem Server einer klinischen Einrichtung, der dem Datenanbieter zugeordnet ist;
wobei ein Patientendatenverzeichnissystem (Patient Data Directory System, PDDS) (100) dazu ausgelegt ist, Datensätze in Bezug auf klinische Daten des Patienten zu speichern, die bei einer Mehrzahl von klinischen Einrichtungen (130) gespeichert sind,
wobei die Datensätze Metadaten umfassen, die den klinischen Daten bei jeder der Mehrzahl von klinischen Einrichtungen zugeordnet sind,
wobei die Metadaten (180) eine Identifikation eines Orts (182) der klinischen Daten bei jeder der Mehrzahl von klinischen Einrichtungen und eine einrichtungsspezifische Pseudo-ID (Site-Specific Pseudo-ID, SPID) (184) des Patienten beinhalten,
wobei die Datenanbietervorrichtung das Kommunikationssystem verwendet, um die klinischen Daten des Patienten in einem Server einer klinischen Einrichtung zu speichern und entsprechende Metadaten als Reaktion zu empfangen, und
wobei die Datenanbietervorrichtung das Kommunikationssystem verwendet, um eine Datensatzaktualisierungsanforderung einschließlich der Metadaten an den Patienten zur nachfolgenden Einreichung an das PDDS zu übermitteln, und wenn der Patient ein neuer Patient ist, Patientenverifizierungsinformationen an das PPDS zu übermitteln, um den Patienten im PDDS darzustellen und jeden der Datensätze des Patienten zu verknüpfen, wobei die Patientenverifizierungsinformationen einen oder mehrere Schlüssel verwenden, um den Patienten im PDDS darzustellen.

19. Datenanforderervorrichtung, umfassend:
ein Kommunikationssystem zum Kommunizieren mit einem Patienten (110) und einer Mehrzahl von klinischen Einrichtungen (130);
wobei der Patient Zugriff auf ein Patientendatenverzeichnissystem (Patient Data Directory System, PDDS) (100) hat, das dazu ausgelegt ist, Datensätze in Bezug auf klinische Daten des Patienten, die bei der Mehrzahl von klinischen Einrichtungen gespeichert sind, und Patientenverifizierungsinformationen zu speichern, die einen oder mehrere Schlüssel verwenden, um den Patienten im PDDS darzustellen und jeden der Mehrzahl von Datensätzen des Patienten zu verknüpfen,
wobei die Datensätze Metadaten umfassen, die den klinischen Daten bei jeder der Mehrzahl von klinischen Einrichtungen zugeordnet sind,
wobei die Metadaten (180) eine Identifikation eines Orts (182) der klinischen Daten bei jeder der Mehrzahl von klinischen Einrichtungen und eine einrichtungsspezifische Pseudo-ID (Site-Specific Pseudo-ID, SPID) (184) des Patienten beinhalten,
wobei die Datenanforderervorrichtung mit einem Authentifizierungstoken versehen ist,
wobei die Datenanforderervorrichtung das Kommunikationssystem verwendet, um eine Datenzugriffsanforderung für alle oder einen ausgewählten Satz von Datensätzen an den Patienten zu übermitteln,
wobei die Datenanforderervorrichtung das Kommunikationssystem verwendet, um die Metadaten, die den klinischen Daten zugeordnet sind, von dem Patienten zu empfangen, und
wobei die Datenanforderervorrichtung das Kommunikationssystem verwendet, um basierend auf den Metadaten und durch Bereitstellen des Authentifizierungstokens an eine entsprechende klinische Einrichtung auf die klinischen Daten des Patienten zuzugreifen, die allen oder dem ausgewählten Satz von Datensätzen zugeordnet sind, die bei der Mehrzahl von klinischen Einrichtungen gespeichert sind.

20. Computerimplementiertes Verfahren (700) zum Verwalten des Zugriffs auf klinische Datensätze eines
Patienten, umfassend:
Speichern (710) einer Mehrzahl von Datensätzen, die den Zugriff auf die klinischen Datensätze des Patienten erleichtern,
wobei die Mehrzahl von Datensätzen Metadaten umfasst, die klinischen Daten des Patienten zugeordnet sind, die bei einer Mehrzahl von klinischen Einrichtungen gespeichert sind,
wobei die Metadaten jedes Datensatzes eine Identifikation einer klinischen Einrichtung der Mehrzahl von klinischen Einrichtungen und eine einrichtungsspezifische Pseudo-ID (Site-Specific Pseudo-ID, SPID), die dem Patienten bei der klinischen Einrichtung zugeordnet ist, umfassen,
wobei die einrichtungsspezifische Pseudo-ID erforderlich ist, um auf die klinischen Daten bei der klinischen Einrichtung zuzugreifen,
Empfangen (720) einer Datensatzzugriffsanforderung von einem Benutzer,
Verifizieren (730), dass der Benutzer der Patient ist, der den Zugriff auf die Metadatendatensätze kontrolliert,
wenn der Benutzer als der Patient verifiziert wird (740) und der Zugriff auf die klinischen Daten des Patienten für einen Datenanforderer durch den Patienten genehmigt wird, Bereitstellen (750, 760) der Metadaten, die in der Datensatzzugriffsanforderung angefordert werden, an den Patienten; und
Übermitteln der Metadaten vom Patienten an den Datenanforderer, um dem Datenanforderer zu ermöglichen, auf die klinischen Daten des Patienten bei der entsprechenden klinischen Einrichtung basierend auf der SPID des Patienten bei der entsprechenden klinischen Einrichtung zuzugreifen.

21. Nicht-transitorisches computerlesbares Medium, umfassend ein Programm, das bei Ausführung
durch ein Verarbeitungssystem bewirkt, dass das Verarbeitungssystem das Verfahren nach Anspruch 20 durchführt.

## Revendications

1. Système de répertoire de données de patient (PDDS) (100) comprenant :
une pluralité d'enregistrements associés à un patient (110),
des informations de vérification de patient (160) qui utilisent une ou plusieurs clés pour représenter le patient dans le PDDS et pour relier chacun de la pluralité d'enregistrements du patient, et
un système de commande d'accès qui commande l'accès à la pluralité d'enregistrements,
l'accès à la pluralité d'enregistrements étant commandé par le patient,
le patient pouvant approuver ou refuser l'accès à des données cliniques du patient par chacun d'une pluralité de demandeurs de données (120), chacun de la pluralité de demandeurs de données étant doté d'un jeton d'authentification,
la pluralité d'enregistrements comprenant des métadonnées qui sont associées à des données cliniques du patient qui sont stockées au niveau d'une pluralité de sites cliniques (130),
chaque site clinique identifiant le patient au moyen d'un pseudo-identifiant spécifique à un site (SPID) du patient,
les métadonnées (180) de chaque enregistrement identifiant un emplacement (182) des données cliniques du patient au niveau d'un site clinique correspondant, et le SPID (184) du patient au niveau du site clinique correspondant,
le système de commande d'accès comprenant un processus de vérification d'identité pour vérifier qu'un utilisateur du système est le patient qui commande l'accès aux enregistrements de métadonnées, et
si l'utilisateur est vérifié comme étant le patient et que l'accès aux données est approuvé pour le demandeur de données, les métadonnées de tous les enregistrements ou d'un ensemble sélectionné d'enregistrements étant communiquées depuis le PDDS, par l'intermédiaire du patient, au demandeur de données afin de permettre au demandeur de données d'accéder aux données cliniques du patient associées auxdits enregistrements au niveau du site clinique correspondant sur la base du SPID du patient au niveau du site clinique correspondant et en fournissant le jeton d'authentification au site clinique correspondant.

2. PDDS (100) selon la revendication 1,
une ou plusieurs clés étant utilisées pour représenter le patient dans le PDDS et pour relier chacun de la pluralité d'enregistrements du patient,
la ou les clés comprenant une ou plusieurs clés aléatoires fournies par le patient et stockées dans le PDDS,
la ou les clés ne révélant pas d'informations d'identification du patient, et
le mécanisme de vérification utilisant un protocole cryptographique basé sur la ou les clés.

3. PDDS (100) selon la revendication 2, le PDDS permettant au patient, après avoir satisfait à la vérification d'identité, de changer la ou les clés qui sont utilisées pour représenter le patient dans le PDDS et pour relier chacun de la pluralité d'enregistrements du patient.

4. PDDS (100) selon la revendication 1,
le mécanisme de vérification comprenant un protocole cryptographique qui requiert une configuration unique et des authentifications ultérieures par défi-réponse,
la configuration unique requérant que le patient fournisse une clé publique et une clé privée d'une paire de clés publique/privée, et
la clé privée étant stockée par le patient et la clé publique étant stockée dans le PDDS par l'intermédiaire d'un fournisseur de données qui enregistre un nouveau SPID dans le PDDS pour le patient.

5. PDDS (100) selon la revendication 1,
le fournisseur de données fournissant le jeton d'authentification au PDDS pour enregistrer un nouveau SPID pour le patient au niveau du PDDS.

6. PDDS (100) selon la revendication 1,
un fournisseur de données fournissant les métadonnées associées aux données cliniques du patient,
le fournisseur de données envoyant au patient une demande de mise à jour d'enregistrement comprenant les métadonnées,
le patient approuvant ou refusant la demande, et
si la demande est approuvée, le patient chiffrant les métadonnées pour créer des métadonnées chiffrées par le patient qui sont stockées dans le PDDS.

7. PDDS (100) selon la revendication 1,
les métadonnées étant chiffrées par le patient pour former des métadonnées chiffrées par le patient qui sont stockées au niveau du PDDS, et
le PDDS comprenant un système de rechiffrement par mandataire qui transforme les métadonnées chiffrées par le patient en métadonnées rechiffrées qui peuvent être déchiffrées par le demandeur de données.

8. PDDS (100) selon la revendication 1,
les métadonnées étant chiffrées par le patient pour former des métadonnées chiffrées par le patient qui sont stockées au niveau du PDDS, et
le PDDS envoyant les métadonnées chiffrées par le patient au patient pour communication ultérieure des métadonnées au demandeur de données.

9. PDDS (100) selon la revendication 1,
le SPID contenu dans les métadonnées qui sont communiquées au demandeur de données étant chiffré à l'aide d'une clé cryptographique appartenant au site clinique correspondant, et
le demandeur de données envoyant le SPID chiffré au site clinique correspondant, le SPID pouvant ensuite être déchiffré au besoin par le site clinique.

10. PDDS (100) selon la revendication 9,
un site clinique possédant plusieurs clés cryptographiques, identifiées chacune par un identifiant de clé unique,
les métadonnées comportant l'identifiant de clé et le SPID chiffré à l'aide de la clé référencée par l'identifiant de clé, et
l'identifiant de clé et le SPID chiffré étant communiqués au demandeur de données afin d'identifier la clé utilisée pour le chiffrement par le site clinique.

11. PDDS (100) selon la revendication 10, le PDDS permettant de remplacer l'identifiant de clé et le SPID qui est chiffré à l'aide de la clé référencée par l'identifiant de clé par un nouvel identifiant de clé et le SPID qui est chiffré à l'aide du nouvel identifiant de clé.

12. PDDS (100) selon la revendication 1, le PDDS comportant une pluralité d'enregistrements de métadonnées pour une pluralité de patients.

13. PDDS (100) selon la revendication 1,
le patient fournissant un accès sélectif aux métadonnées à chaque demandeur de données, et
l'accès sélectif étant basé sur des informations annexes contenues dans les métadonnées,
les informations annexes comprenant un ou plusieurs éléments parmi :
une région anatomique associée aux données cliniques,
une période liée aux données cliniques, et
un diagnostic associé aux données cliniques.

14. PDDS (100) selon la revendication 1,
une paire de clés publique-privée étant associée au patient,
la clé publique de la paire de clés publique-privée étant communiquée au PDDS, et
le système PDDS permettant au patient, après avoir satisfait à la vérification d'identité, de changer la clé publique dans le PDDS pour une nouvelle clé publique correspondant à une nouvelle paire de clés publique-privée du patient.

15. Dispositif patient destiné à être utilisé par un patient (110), comprenant :
un système de communication pour communiquer avec un demandeur de données (120) et un système de répertoire de données de patient (PDDS) (100), et
une interface utilisateur ;
le PDDS étant configuré pour stocker des enregistrements liés à des données cliniques du patient stockées au niveau d'une pluralité de sites cliniques (130) et des informations de vérification de patient qui utilisent une ou plusieurs clés pour représenter le patient dans le PDDS et pour relier chacun de la pluralité d'enregistrements du patient,
les enregistrements comprenant des métadonnées associées aux données cliniques au niveau de chacun de la pluralité de sites cliniques,
les métadonnées (180) comprenant une identification d'un emplacement (182) des données cliniques au niveau de chacun de la pluralité de sites cliniques, et un pseudo-identifiant spécifique à un site (SPID) (184) du patient,
lorsque le dispositif patient reçoit du demandeur de données une demande d'accès aux enregistrements portant sur tous les enregistrements ou sur un ensemble sélectionné d'enregistrements :
le dispositif patient, par l'intermédiaire de l'interface utilisateur, présentant la demande et recevant du patient une réponse d'approbation ou de refus,
si la demande est approuvée :
le dispositif patient utilisant le système cryptographique pour accéder aux enregistrements de métadonnées du patient au niveau du PDDS,
le dispositif patient utilisant le système de communication pour communiquer la demande d'accès aux enregistrements au PDDS,
le dispositif patient utilisant le système de communication pour recevoir, en réponse à la demande d'accès aux enregistrements, les enregistrements de métadonnées du patient en provenance du PDDS, et
le dispositif patient utilisant le système de communication pour communiquer les métadonnées sélectionnées par le patient au demandeur de données, afin de faciliter l'accès, par le demandeur de données, aux données cliniques du patient stockées au niveau de la pluralité de sites cliniques et associées à tous les enregistrements ou à l'ensemble sélectionné d'enregistrements.

16. Dispositif patient selon la revendication 15, comprenant un système cryptographique,
lorsque le dispositif patient reçoit une demande de mise à jour d'enregistrement en provenance du fournisseur de données :
le dispositif patient, par l'intermédiaire de l'interface utilisateur, présentant la demande au patient et recevant du patient une réponse d'approbation ou de refus,
le dispositif patient utilisant le système cryptographique pour accéder au PDDS, et
le dispositif patient utilisant le système de communication pour communiquer les métadonnées au PDDS.

17. Dispositif patient selon la revendication 15, le dispositif patient comprenant le PDDS (100).

18. Dispositif fournisseur de données destiné à être utilisé par un fournisseur de données, comprenant :
un système de communication pour communiquer avec un patient (110) et un serveur de site clinique associé au fournisseur de données ;
un système de répertoire de données de patient (PDDS) (100) étant configuré pour stocker des enregistrements liés à des données cliniques du patient stockées au niveau d'une pluralité de sites cliniques (130),
les enregistrements comprenant des métadonnées associées aux données cliniques au niveau de chacun de la pluralité de sites cliniques,
les métadonnées (180) comprenant une identification d'un emplacement (182) des données cliniques au niveau de chacun de la pluralité de sites cliniques, et un pseudo-identifiant spécifique à un site (SPID) (184) du patient, le dispositif fournisseur de données utilisant le système de communication pour stocker les données cliniques du patient dans un serveur de site clinique et recevoir des métadonnées correspondantes en réponse, et
le dispositif fournisseur de données utilisant le système de communication pour communiquer au patient une demande de mise à jour d'enregistrement comprenant les métadonnées en vue d'une soumission ultérieure au PDDS, et, si le patient est un nouveau patient, pour communiquer des informations de vérification de patient au PDDS afin de représenter le patient dans le PDDS et de relier chacun des enregistrements du patient, les informations de vérification de patient utilisant une ou plusieurs clés pour représenter le patient dans le PDDS.

19. Dispositif demandeur de données, comprenant :
un système de communication pour communiquer avec un patient (110) et une pluralité de sites cliniques (130) ;
le patient ayant accès à un système de répertoire de données de patient (PDDS) (100) qui est configuré pour stocker des enregistrements liés à des données cliniques du patient stockées au niveau de la pluralité de sites cliniques et des informations de vérification de patient qui utilisent une ou plusieurs clés pour représenter le patient dans le PDDS et pour relier chacun de la pluralité d'enregistrements du patient,
les enregistrements comprenant des métadonnées associées aux données cliniques au niveau de chacun de la pluralité de sites cliniques,
les métadonnées (180) comprenant une identification d'un emplacement (182) des données cliniques au niveau de chacun de la pluralité de sites cliniques, et un pseudo-identifiant spécifique à un site (SPID) (184) du patient, le dispositif demandeur de données étant doté d'un jeton d'authentification,
le dispositif demandeur de données utilisant le système de communication pour communiquer au patient une demande d'accès aux données portant sur tous les enregistrements ou sur un ensemble sélectionné d'enregistrements,
le dispositif demandeur de données utilisant le système de communication pour recevoir, en provenance du patient, les métadonnées associées aux données cliniques, et
le dispositif demandeur de données utilisant le système de communication pour accéder aux données cliniques du patient stockées au niveau de la pluralité de sites cliniques et associées à tous les enregistrements ou à l'ensemble sélectionné d'enregistrements, sur la base des métadonnées et en fournissant le jeton d'authentification à un site clinique correspondant.

20. Procédé (700) mis en œuvre par ordinateur pour la gestion de l'accès à des dossiers cliniques d'un patient, comprenant :
le stockage (710) d'une pluralité d'enregistrements qui facilitent l'accès aux dossiers cliniques du patient,
la pluralité d'enregistrements comprenant des métadonnées qui sont associées à des données cliniques du patient qui sont stockées au niveau d'une pluralité de sites cliniques,
les métadonnées de chaque enregistrement comprenant une identification d'un site clinique de la pluralité de sites cliniques et un pseudo-identifiant spécifique à un site (SPID) associé au patient au niveau du site clinique,
le pseudo-identifiant spécifique à un site étant nécessaire pour accéder aux données cliniques au niveau du site clinique,
la réception (720) d'une demande d'accès aux enregistrements en provenance d'un utilisateur,
la vérification (730) de ce que l'utilisateur est le patient qui commande l'accès aux enregistrements de métadonnées,
si l'utilisateur est vérifié (740) comme étant le patient et que l'accès aux données cliniques du patient est approuvé pour un demandeur de données par le patient, la fourniture (750, 760) au patient des métadonnées demandées dans la demande d'accès aux enregistrements ; et
la communication des métadonnées, en provenance du patient, au demandeur de données pour permettre au demandeur de données d'accéder aux données cliniques du patient au niveau du site clinique correspondant sur la base du SPID du patient au niveau du site clinique correspondant.

21. Support non transitoire lisible par ordinateur comprenant un programme qui, lorsqu'il est exécuté par un système de traitement, amène le système de traitement à réaliser le procédé selon la revendication 20.
